# EUROPEAN PATENT SPECIFICATION

(11) **EP 1 369 119 B1**
(45) Date of publication and mention of the grant of the patent: **17.12.2008**
(21) Application number: 02705197.8
(22) Date of filing: 14.03.2002
(51) Int. Cl.: A61K 31/728, A61P 43/00, A61P 17/00, A61P 27/02, A61P 37/08, A61P 37/04, A61P 3/10, A61P 25/00, A61P 29/00, A61P 19/00, A61P 17/06, A61P 13/12, C08B 37/08

(54) **IL-12 EXPRESSION CONTROLLING AGENTS**
MITTEL ZUR STEURUNG DER EXPRESSION VON IL-12
AGENTS DE REGULATION D'EXPRESSION D'IL-12

(30) Priority: 15.03.2001 JP 2001074077; 15.03.2001 JP 2001074078
(43) Date of publication of application: 10.12.2003
(73) Proprietor: SEIKAGAKU CORPORATION, Chiyoda-ku, Tokyo 100-0005 (JP)
(72) Inventor: ASARI, Akira, Iruma-shi, Saitama 358-0053 (JP); KURIHARA, Hitoshi, Musashimurayama-shi, Tokyo 208-0003 (JP)
(74) Representative: HOFFMANN EITLE
(86) International application number: PCT/JP2002/002433
(87) International publication number: WO 2002/074318

(56) References cited:
- TERMEER C C ET AL: "Oligosaccharides of hyaluronan are potent activators of dendritic cells" JOURNAL OF IMMUNOLOGY 15 AUG 2000 UNITED STATES, vol. 165, no. 4, 15 August 2000 (2000-08-15), pages 1863-1870, XP002300337 ISSN: 0022-1767
- DATABASE EMBASE [Online] ELSEVIER SCIENCE PUBLISHERS, AMSTERDAM, NL; 1994, NAKAJIMA H ET AL: "Acute toxicity study of high molecular weight sodium hyaluronate (NRD101) in rats" XP002300338 Database accession no. EMB-1994166181 & JAPANESE PHARMACOLOGY AND THERAPEUTICS 1994 JAPAN, vol. 22, no. SUPPL. 3, 1994, pages 7-15, ISSN: 0386-3603
- DATABASE EMBASE [Online] ELSEVIER SCIENCE PUBLISHERS, AMSTERDAM, NL; 1994, TAMOTO K ET AL: "High molecular weight hyaluronic acids inhibit interleukin-1-induced prostaglandin E2 generation and prostaglandin E2-elicited cyclic AMP accumulation in human rheumatoid arthritic synovial cells" XP002300339 Database accession no. EMB-1994345904 & JAPANESE JOURNAL OF RHEUMATOLOGY 1994 NETHERLANDS, vol. 5, no. 3, 1994, pages 227-236, ISSN: 0169-1163
- HODGE-DUFOUR JENNIFER ET AL.: 'Induction of IL-12 and chemokines by hyaluronan requires adhesion-dependent priming of resident but not elicited macrophages' J. IMMUNOL. vol. 159, no. 5, 1997, pages 2492 - 2500, XP002951519

## Description

### TECHNICAL FIELD

The present invention relates to certain uses of an interleukin 12 (IL-12) expression controlling agent for oral administration, which comprises hyaluronan or a pharmaceutically acceptable salt thereof as an active ingredient.

Furthermore, the present invention relates to certain uses of an inhibitor of interleukin 12 (IL-12) expression for oral administration, which comprises hyaluronan or a pharmaceutically acceptable salt thereof having a weight average molecular weight of from 600,000 to 3,000,000 as an active ingredient.

Moreover, the present invention relates to certain uses of an enhancer of interleukin 12 (IL-12) expression for oral administration, which comprises hyaluronan or a pharmaceutically acceptable salt thereof having a weight average molecular weight of from 50,000 to 400,000 as an active ingredient.

### BACKGROUND OF THE INVENTION

It is known that IL-12 is a cytokine of a 70 kD glycoprotein (p70) in which two polypeptide chains of 35 kD (p35) and 40 kD (p40) are linked to each other, and plays a central role in the regulation of immunological functions in the living body (Cytokine, edited by Shinpei Kasakura, revised new edition of the Second Edition, pp. 207-225, published by Nihon Igakukan, Inc., June 29, 1997).

It is known that since IL-12 acts on the differentiation induction of T helper 1 cell subset (Th1) of helper T cell, it acceleratively acts on the advance of morbid states in autoimmune diseases relating to activation of Th1.

J. Immunol., 165(4), 1863-1870 (2000) discloses that an oligosaccharide of hyaluronan increases IL-12 production of dendritic cells, but hyaluronan having a molecular weight of from 80,000 to 200,000 or a molecular weight of from 1,000,000 to 600,000 does not have such an activity. However, it neither discloses nor suggests the activity of hyaluronan to inhibit IL-12 production.

On the other hand, it is known that IL-12 plays a preventive role against microbial infection and shows an antitumor effect as a cytokine relating to the differentiation induction and the like of T helper 1 cell subset (Th1) of helper T cell.

J. Immunol., 159(5), 2492-2500 (1997) discloses that hyaluronan having a molecular weight of about 280,000 induced IL-12 production of macrophage *in vitro.*

Contrary to this, J. Immunol., 165(4), 1863-1870 (2000) discloses that an oligosaccharide of hyaluronan (about from 4 sugars to 14 sugars) increases IL-12 production of dendritic cells, but hyaluronan having a molecular weight of from 80,000 to 200,000 or a molecular weight of from 1,000,000 to 600,000 does not have such an activity.

Thus, various *in vitro* tests have been carried out regarding the acceleration of IL-12 production by hyaluronan, but information about which molecular weight of hyaluronan is effective for the acceleration of IL-12 production varies depending on literatures and is still unclear. In addition, all of these literatures are *in vitro* approach, and there is no approach actually carried out by taking note of its application as medicaments, particularly a "medicament for oral administration" which can be administered most conveniently.

An object of the present invention is to provide an IL-12 expression controlling agent, an inhibitor of IL-12 expression, an enhancer of IL-12 expression for oral administration and an IL-12 expression controlling agent for oral administration, which contain hyaluronan as an active ingredient.

### DISCLOSURE OF THE INVENTION

As a result of intensive studies carried out in order to solve the problems, the present inventors have found that hyaluronan controls IL-12 expression, specifically, hyaluronan having a specific weight average molecular weight has an activity to inhibit IL-12 expression, and that an inhibitor of IL-12 expression can be provided by using it. Also, the inventors have found that the above effect can also be obtained by oral administration of hyaluronan.

Furthermore, the inventors have found that hyaluronan having a specific weight average molecular weight has an activity to enhance IL-12 expression, and that the activity can also be obtained by oral administration to thereby find that an enhancer of IL-12 expression for oral administration can be provided by using it. The present invention has been completed based on these findings.

That is, the present invention provides certain uses of an IL-12 expression controlling agent, which comprises hyaluronan or a pharmaceutically acceptable salt thereof as an active ingredient (hereinafter referred to as "controlling agent of the invention"). The controlling agent of the invention can inhibit or enhance IL-12 expression. In particular, the present invention provides the following:
1. Use of hyaluronan or a pharmaceutically acceptable salt thereof having a weight average molecular weight of from 600,000 to 3,000,000 for preparing a medicament for the treatment or prevention of autoimmune diseases relating to activation of Th1, wherein the medicament is used for oral administration.
2. Use of hyaluronan or a pharmaceutically acceptable salt thereof having a weight average molecular weight of from 600,000 to 3,000,000 for preparing a medicament for the treatment or prevention of contact dermatitis, autoimmune uvea retinitis, allergic cerebrospinal meningitis, insulin-dependent diabetes mellitus, diabetes mellitus, Hashimoto disease, multiple sclerosis, rheumatic arthritis, Sjögren syndrome, Crohn disease, sarcoidosis, psoriasis, lipopolysaccharide-induced hepatic necrosis, crescentic glomerulonephritis, or systemic lupus erythematosus, wherein the medicament is used for oral administration.
3. The use according to embodiment 2, wherein the medicament is for the treatment or prevention of rheumatic arthritis or Crohn disease.
4. The use according to any one of embodiments 1 to 3, wherein the hyaluronan or the pharmaceutically acceptable salt thereof has a weight average molecular weight of from 600,000 to 1,200,000.
5. The use according to any one of embodiments 1 to 4, wherein the hyaluronan or the pharmaceutically acceptable salt thereof has a weight average molecular weight of from 700,000 to 1,200,000.
6. The use according to any one of embodiments 1 to 5, wherein the hyaluronan or the pharmaceutically acceptable salt thereof has a weight average molecular weight of from 700,000 to 1,100,000.
7. The use according to any one of embodiments 1 to 6, wherein the hyaluronan or the pharmaceutically acceptable salt thereof has a weight average molecular weight of from 800,000 to 1,100,000.
8. The use according to any one of embodiments 1 to 7, wherein the hyaluronan or the pharmaceutically acceptable salt thereof has a weight average molecular weight of from 800,000 to 1,000,000.
9. The use according to any one of embodiments 1 to 8, wherein the hyaluronan or the pharmaceutically acceptable salt thereof has a weight average molecular weight of from 800,000 to 950,000.
10. The use according to any one of embodiments 1 to 9, wherein the hyaluronan or the pharmaceutically acceptable salt thereof has a weight average molecular weight of from 800,000 to 900,000.
11. Use of hyaluronan or a pharmaceutically acceptable salt thereof having a weight average molecular weight of from 50,000 to 400,000 for preparing a medicament for the treatment or prevention of diseases caused by microbial infection, viral diseases or cancers for oral administration.
12. The use according to embodiment 11, wherein the hyaluronan or the pharmaceutically acceptable salt thereof has a weight average molecular weight of from 100, 000 to 300,000.
13. Hyaluronan or a pharmaceutically acceptable salt thereof having a weight average molecular weight of from 600,000 to 3,000,000 for use in the treatment or prevention of autoimmune diseases relating to activation of Th1, wherein the hyaluronan or pharmaceutically acceptable salt thereof is used for oral administration.
14. Hyaluronan or a pharmaceutically acceptable salt thereof having a weight average molecular weight of from 600,000 to 3,000,000 for use in the treatment or prevention of contact dermatitis, autoimmune uvea retinitis, allergic cerebrospinal meningitis, insulin-dependent diabetes mellitus, diabetes mellirus, Hashimoto disease, multiple sclerosis, rheumatic arthritis, Sjögren syndrome, Crohn disease, sarcoidosis, psoriasis, lipopolysaccharide-induced hepatic necrosis, crescentic glomerulonephritis, or systemic lupus erythematosus, wherein the hyaluronan or pharmaceutically acceptable salt thereof is used for oral administration.
15. The hyaluronan or pharmaceutically acceptable salt thereof according to embodiment which is for the treatment or prevention of rheumatic arthritis or Crohn disease.
16. The hyaluronan or pharmaceutically acceptable salt thereof according to any one of embodiments 13 to 15, wherein the hyaluronan or the pharmaceutically acceptable salt thereof has a weight average molecular weight of from 600,000 to 1,200,000.
17. The hyaluronan or pharmaceutically acceptable salt thereof according to any one of embodiments 13 to 16, wherein the hyaluronan or the pharmaceutically acceptable salt thereof has 2 weight average molecular weight of from 700.000 to 1,200,000.
18. The hyaluronan or pharmaceutically acceptable salt thereof according to any one of embodiments 13 to 17, wherein the hyaluronan or the pharmaceutically acceptable salt thereof has a weight average molecular weight of from 700,000 to 1,100,000.
19. The hyaluronan or pharmaceutically acceptable salt thereof according to any one of embodiments 13 to 18, wherein the hyaluronan or the pharmaceutically acceptable salt thereof has a weight average molecular weight of from 800,000 to 1,100,000.
20. The hyaluronan or pharmaceutically acceptable salt thereof according to any one of embodiments 13 to 19, wherein the hyaluronan or the pharmaceutically acceptably salt thereof has a weight average molecular weight of from 800,000 to 1,000,000.
21. The hyaluronan or pharmaceutically acceptable salt thereof according to any one of embodiments 13 to 20, wherein the hyaluronan or the pharmaceutically acceptable salt thereof has a weight average molecular weight of from 800,000 to 950,000.
22. The hyaluronan or pharmaceutically acceptable salt thereof according to any one of embodiments 13 to 21, wherein the hyaluronan or the pharmaceutically acceptable salt thereof has a weight average molecular weight of from 800,000 to 900,000.
23. Hyaluronan or a pharmaceutically acceptable salt thereof having a weight average molecular weight of from 50,000 to 400,000 for use in the treament or prevention of diseases caused by microbial infection viral diseases or cancers for oral administration.
24. The hyaluronan or pharmaceutically acceptable salt thereof according to embodiment 23, wherein the hyaluronan or the pharmaceutically acceptable salt thereof has a weight average molecular weight of from 100,000 to 300,000.

### BRIEF DESCRIPTION OF THE DRAWINGS

Fig. 1 shows decrease in serum IL-12 by oral administration ofHA.
Fig. 2 shows increase in serum IL-12 by oral administration of HA.

### BEST MODE FOR CARRYING OUT THE INVENTION

### <1> Hyaluronan or pharmaceutically acceptable salt thereof

The origin of the hyaluronan or the pharmaceutically acceptable salt thereof used in the present invention is not particularly limited, and hyaluronan separated and purified from crests, umbilical cords, hyaluronan-producing microorganisms and the like can be used. Particularly, a preparation which is purified to such a high purity that it is substantially free of a substance whose contamination is not allowed as medicaments and food is preferred.

Examples of the pharmaceutically acceptable salt of hyaluronan include pharmaceutically acceptable salts among salts with inorganic base salts such as alkali metal salts (sodium salt, lithium salt, potassium salt, *etc*.), alkaline earth metal salts and ammonium salts and the like, and salts with organic bases such as diethanolamine salts, cyclohexylamine salts and amino acid salts. Among these, sodium hyaluronate is preferable.

When the controlling agent of the invention is used for inhibition of IL-12 expression, the following description on the inhibitor of the invention is similarly applied. Also, when it is used for enhancement of IL-12 expression, the following description on the enhancer of the invention is similarly applied. Accordingly, when the controlling agent of the invention is used for inhibition of IL-12 expression, the following description on the inhibitor of the invention should be referred, and when the controlling agent of the invention is used for the enhancement of IL-12 expression, the following description on the enhancer of the invention should be referred.

In addition, when the oral agent of the invention is used for inhibition of IL-12 expression, the following description on the inhibitor of the invention (a description part regarding oral administration) is similarly applied. Also, when it is used for enhancement of IL-12 expression, the following description on the enhancer of the invention (a description part regarding oral administration) is similarly applied. Accordingly, since the oral preparation of the present invention is used for inhibition of IL-12 expression, the following description on the inhibitor of the invention (a part regarding oral administration) is referred, and when the controlling agent of the invention is used for the enhancement of IL-12 expression, the following description on the enhancer of the invention (a part regarding oral administration) should be referred.

The weight average molecular weight of the hyaluronan or the pharmaceutically acceptable salt thereof used in the inhibitor of the invention is not particularly limited, so long as it is from 600,000 to 3,000,000. As.is shown below in Examples; hyaluronan or a pharmaceutically acceptable salt thereof shows excellent activity for production of IL-12 when the weight average molecular weight is about from 840,000 to 850,000, and shows effects within the above specific weight average molecular weight range. The lower limit of the weight average molecular weight of the hyaluronan or the pharmaceutically acceptable salt thereof used in the inhibitor of the invention is 600,000, preferably 700,000, more preferably 800,000, and the upper limit thereof is 3,000,000, preferably 1,200,000, more preferably 1,100,000, still more preferably 1,000,000, still far more preferably 950,000, and particularly preferably 900,000. The weight average molecular weight of the hyaluronan or the pharmaceutically acceptable salt thereof used in the inhibitor of the invention is most preferably about from 840,000 to 900,000.

The hyaluronan or the pharmaceutically acceptable salt thereof used in the inhibitor of the invention has a limiting viscosity of about from 11.5 to 44 dl/g, preferably about from 11.5 to 20 dl/g, more preferably about from 13.0 to 20 dl/g, still more preferably about from 13.0 to 18.5 dl/g, still far more preferably about from 14.5 to 18.5 dl/g, particularly preferably about from 14.5 to 17.5 dl/g, most preferably about from 14.5 to 16.5 dl/g, far most preferably about from 14.5 to 16 dl/g, ultimately preferably about from 15 to 16 dUg. Among these, one having a limiting viscosity of around 15 dl/g is more preferable.

An inhibitor of IL-12 expression having excellent activity can be obtained by using the above hyaluronan or pharmaceutically acceptable salt thereof.

On the other hand, the weight average molecular weight of the hyaluronan or the pharmaceutically acceptable salt thereof used in the enhancer of the invention is not particularly limited, so long as it is from 50,000 to 400,000. As is described below in Examples, hyaluronan or a pharmaceutically acceptable salt thereof shows excellent activity for IL-12 production when the weight average molecular weight is from 100,000 to 300,000, and shows the effect within the above specific weight average molecular weight range.

The lower limit of the weight average molecular weight of the hyaluronan or the pharmaceutically acceptable salt thereof used in the enhancer of the invention is 50,000 and preferably 100,000, and the upper limit thereof is 400,000 and preferably 300,000.

The above hyaluronan or pharmaceutically acceptable salt thereof used in the enhancer of the invention has a limiting viscosity of about from 2 to 8.5 dl/g, and preferably about from 5 to 7 dl/g.

An enhancer of IL-12 expression for oral administration having excellent activity can be obtained by using the above hyaluronan or pharmaceutically acceptable salt thereof.

Also, the weight average molecular weight of the hyaluronan or the pharmaceutically acceptable salt thereof used in the inhibitor of the invention or the enhancer of the invention can be calculated based on the equation of Laurent et al. (Biochim. Biophys. Acta, 42, 476 (1960)) by measuring its limiting viscosity in accordance with The Pharmacopoeia of Japan, 13th revised edition: General Test Method, Chapter 36, Viscosity Measuring Method.

Also, the upper limit of the endotoxin concentration in the hyaluronan or the pharmaceutically acceptable salt thereof used in the inhibitor of the invention and the enhancer of the invention can be optionally set according to specific use and the like of the inhibitor of the invention and the enhancer of the invention. For example, when it is used as a reagent for use in a test which should be carried out in the absence of an endotoxin or as a medicament or the like which is directly administered into blood vessels, it is preferably 0.3 EU/ml or less when it is made into a solution preparation. Also, when orally administered like the case of the enhancer of the invention, it can be optionally set within such a range that it does not cause problems in carrying out oral administration. In this case, it is preferably 0.3 EU/ml or less when it is made into a solution preparation.

The endotoxin concentration can be measured using any endotoxin measuring method well known to and conventionally used by one skilled in the art, but the Limulus test using a horseshoe crab amoebocyte lysate component is preferable. Also, the EU (endotoxin unit) can be measured and calculated in accordance with Japan Industrial Standard, Provisions for Biochemical Reagents (JIS K8008). In addition, it is preferable that the iron content is 20 ppm or less.

### <2> Dosage form, etc. of the inhibitor of the invention or the enhancer of the invention

The inhibitor of the invention is used as a medicament or the like, it is administered by oral administration. According to this administration method, it can be prepared as solutions suspensions, emulsions, solid preparations for dissolution before use, *etc*.), tablets, capsules, solutions, granules, powders, lipo-forming preparations, gels, sprays, inhalation powders and the like.

The concentration of the hyaluronan or the pharmaceutically acceptable salt thereof in the inhibitor of the invention is not particularly limited, too, and is preferably from 0.5 to 10% (w/v). When the inhibitor of the invention is used for example as solutions for oral administration, the concentration is preferably 0.1% (w/v) or more, and more preferably about from 0.1 to 1% (w/v).

On the other hand, the enhancer of the invention is orally administered for applying to an animal which requires enhancement of IL-12 production. According to the object, subject and the like of oral administration, the enhancer of the invention can be prepared as tablets, capsules, solutions, granules, powders, lipo-forming preparations, inhalation powders and the like.

The concentration of the hyaluronan or the pharmaceutically acceptable salt thereof in the enhancer of the invention is not particularly limited, too, and is preferably from 0.5 to 10% (w/v). For example, when the enhancer of the invention is used as solutions for oral administration, the concentration is preferably 0.5% (w/v) or more, and more preferably about from 0.5 to 2% (w/v).

The inhibitor of the invention or the enhancer of the invention can be prepared using known methods. In addition, in preparing these preparations, other pharmaceutically active components and components generally used in medicaments, such as conventionally used stabilizing agents, emulsifying agents, osmotic pressure adjusting agents, buffer agents, tonicity agents, preservatives, soothing agents, coloring agents, excipients, binders, lubricants, disintegrating agents and the like, can be used, so long as they do not show bad influences on the hyaluronan or the pharmaceutically acceptable salt thereof and also do not show influences on the effects of the present invention.

### <3> Subject of administration, etc. when the inhibitor of the invention or the enhancer of the invention is used for medical treatment

As the animal to which the inhibitor of the invention is administered, vertebral animals, particularly mammals, are preferable, and human is particularly preferable. The inhibitor of the invention can be administered to these animals as a medicament for the purpose of inhibiting IL-12 production. The applicable disease is not limited, so long as it is a disease whose cause of disease is the activation of Th1 in which IL-12 positively acts upon the advance of the condition of the disease. Specific examples of the diseases include contact dermatitis, autoimmune uvea retinitis, allergic cerebrospinal meningitis, insulin-dependent diabetes mellitus, diabetes mellitus, Hashimoto disease, multiple sclerosis, rheumatic arthritis, Sjögren syndrome, Crohn disease, sarcoidosis, psoriasis, lipopolysaccharide-induced hepatic necrosis, crescentic glomerulonephritis, systemic lupus erythematosus and the like. Thus, the inhibitor of the invention also includes a concept as a treating agent of these diseases. When the inhibitor of the invention is used as a medicament, its administration may be not only for the purpose of pure treatment but also for the purpose of prevention, advance inhibition (worsening prevention), alleviation (improvement of symptoms) and the like of diseases.

A formulating amount, a dose per one administration, an administration interval and the like of the hyaluronan or the pharmaceutically acceptable salt thereof in the inhibitor of the invention are not particularly limited, because they are items which should be separately decided according to the administration method, dosage form, using purpose and the like of the inhibitor of the invention and specific symptoms, age, sex, body weight and the like of each patient, but as the clinical dose of the hyaluronan or a pharmaceutically acceptable salt thereof, from 50 to 250 mg per once and from 100 to 500 mg per day, per adult in the case of oral administration. Also, the administration interval of the inhibitor of the invention may be about once a day, and it can be administered by dividing the daily dose into 2 to 3 doses per day. Alternatively, it may be administered once during a period of about from 1 to 3 days.

As the animal to which the enhancer of the invention is administered, vertebral animals, particularly mammals, are also preferable, and human is particularly preferable. The enhancer of the invention can be orally administered to these animals as a medicament for the purpose of enhancing IL-12 production. The applicable disease is not limited, so long as the enhancement of IL-12 production is the object, and examples include diseases caused by microbial infection, viral diseases (AIDS, hepatitis C and the like), tumors (cancers), diseases caused by the reduction of IL-12 production, and the like. Thus, the enhancer of the invention also includes a concept as a treating agent of these diseases. When the enhancer of the invention is used as a medicament, its administration may be not only for the purpose of pure treatment but also for the purpose of prevention, advance inhibition (worsening prevention), alleviation (improvement of symptoms) and the like of diseases.

A formulating amount, a dose per one administration, an administration interval and the like of the hyaluronan or the pharmaceutically acceptable salt thereof in the enhancer of the invention are not particularly limited, because they are items which should be separately decided according to the administration method, dosage form, using purpose and the like of the enhancer of the invention and specific symptoms, age, sex, body weight and the like of each patient, but as the clinical dose of the hyaluronan or a pharmaceutically acceptable salt thereof, from 50 to 250 mg per once and from 100 to 500 mg per day, per adult in the case of oral administration. Also, the administration interval of the enhancer of the invention may be about once a day, and it can be administered by dividing the daily dose into 2 to 3 doses per day. Alternatively, it may be administered once during a period of about from 1 to 3 days.

Also, hyaluronan or pharmaceutically acceptable salts thereof have already been used in medicaments, cosmetics, food and the like and it is known that they have high safety.

### Examples

Examples of the present invention are specifically described below. However, technical scope of the present invention is not limited thereto.

### Example 1. Inhibition of IL-12 expression

### <1> Material to be tested

The materials to be tested used in this example are as follows.
- Phosphate buffered saline (PBS)
- Sodium hyaluronate (weight average molecular weight: 840,000; limiting viscosity: 15.0 dl/g). Hereinafter, this sodium hyaluronate is referred to as HA.

HA was used by dissolving it in PBS to a predetermined concentration according to each of the following drug efficacy pharmacological tests. The endotoxin concentration after dissolving in PBS was 0.3 EU/ml or less in each case, and the iron content was 20 ppm or less in each case.

### <2> Drug efficacy pharmacological test

### (1) Reduction of IL-12 p40 production in macrophage

Into the abdominal cavity of a 4-weeks-old male C57BL/6 mouse (Charles River Japan, Inc.), 5 to 7 ml of ice-cooled Hanks' solution was injected and recovered 5 minutes thereafter to collect commonly existing peritoneal cells. The thus collected cells were dispensed in a 96 well flat bottom plate and cultured using DMEM medium at 37°C overnight under 5% CO₂. After the culturing suspended cells were removed and the adhered cells were used in the test as the peritoneal macrophage.

In order to stimulate IL-12 production, a lipopolysaccharide (LPS) (List Biological Laboratories, Inc.) was added to media containing varied concentrations of HA, to give a final concentration of 1 µg/ml, and the cells were treated for 24 hours. Thereafter, the concentration of IL-12 (p40) in the cell culture supernatant was measured using Mouse IL-12 (p40) ELISA Kit (manufactured by Endogen) to evaluate IL-12 production. In this case, the test was carried out three times and the average value was calculated. The results are shown below.

**Table 1**

| No treatment | LPS alone | LPS + 10 µg/ml HA | LPS + 100 µg/ml HA |
|---|---|---|---|
| 0.0 | 26.8 | 21.3 | 18.5 |
| (unit: pg/ml) | | | |

Based on this result, it was shown that a high molecular weight sodium hyaluronate having a weight average molecular weight of about 840,000 inhibits IL-12 production.

Separately from this, a similar test was carried out using sodium hyaluronate (100 µg/ml) having other weight average molecular weight (600,000 or 2,700,000; limiting viscosity 11.5 dl/g or 40.5 dl/g). The test was carried out three times and the average value was calculated.

As a result, the concentration of IL-12 (p40) in the cell culture supernatant was 65.1 pg/ml (4.6) in the case of LPS alone, 56.0 pg/ml (5.1) in the case of LPS + sodium hyaluronate having a weight average molecular weight of 600,000, and 60.0 pg/ml (7.7) in the case ofLPS + sodium hyaluronate having a weight average molecular weight of 2,700,000. Also, the numeral in parentheses indicates a standard deviation.

Based on this result, each of the sodium hyaluronate samples did not inhibit IL-12 production significantly but showed a tendency to inhibit it though slightly. In addition, this tendency was slightly high in the case of the molecular weight of 600,000.

### (2) Reduction of serum IL-12 by HA administration

Four-weeks-old male C57BL/6 mice of 4 weeks of age (Charles River Japan, Inc.) were purchased and divided into PBS administration group (n = 5) and HA administration group (n = 5).

A PBS solution containing 10 mg/ml HA was orally administered at a dose of 80 mg/kg body weight per once. The oral administration was carried out in the usual way using sterilized 1 ml capacity disposable syringes and sterilized oral sounds. The administration was carried out twice a day continuously for 2 weeks.

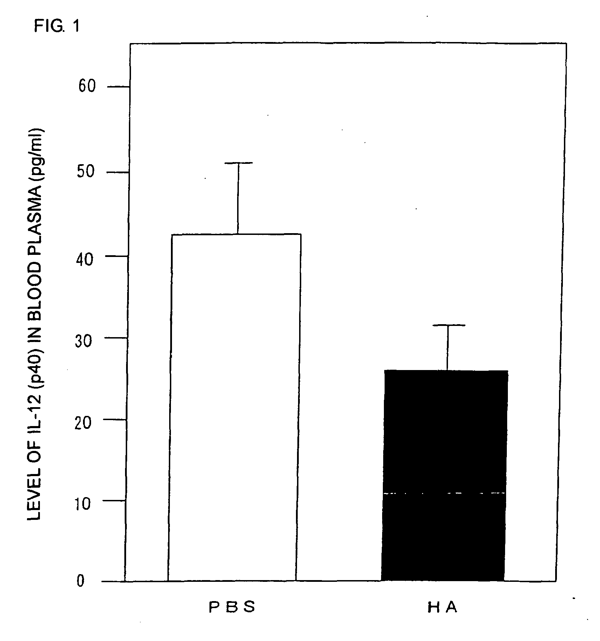

After completion of the administration, blood samples were collected from the orbital venous plexus, and concentration of IL-12 (p40) in blood plasma was measured using Mouse IL-12 (p40) ELISA Kit (manufactured by Endogen) to calculate its average value and standard deviation. The results are shown in Fig. 1.

Based on this result, it was shown that IL-12 production can be inhibited also by oral administration of a high molecular weight sodium hyaluronate having a weight average molecular weight of about 840,000.

### Example 2. Enhancement of IL-12 expression

### <1> Material to be tested

The materials to be tested used in this example are as follows.
- Phosphate buffered saline (PBS)
- Sodium hyaluronate (weight average molecular weight: 100,000 to 200,000; limiting viscosity: 3 dl/g to 5 dl/g). Hereinafter, this sodium hyaluronate is called "HA10-20".
- Sodium hyaluronate (weight average molecular weight: about 300,000; limiting viscosity: about 7 dk/g). Hereinafter, this sodium hyaluronate is called "HA30".

HA was used by dissolving it in PBS to a predetermined concentration according to each of the following drug efficacy pharmacological tests. The endotoxin concentration after dissolving in PBS was 0.3 EU/ml or less in each case, and the iron content was 20 ppm or less in each case.

### <2> Drug efficacy pharmacological test

### (1) Increase of serum IL-12 by HA10-20 administration

Four-weeks-old male C57BL/6 mice (Charles River Japan, Inc.) were purchased and divided into PBS administration group (n = 5) and HA10-20 administration group (n = 5).

A PBS solution containing 10 mg/ml HA10-20 was orally administered at a dose of 80 mg/kg body weight per once. The oral administration was carried out in the usual way using sterilized 1 ml capacity disposable syringes and sterilized oral sounds. The administration was carried out twice a day continuously for 2 weeks.

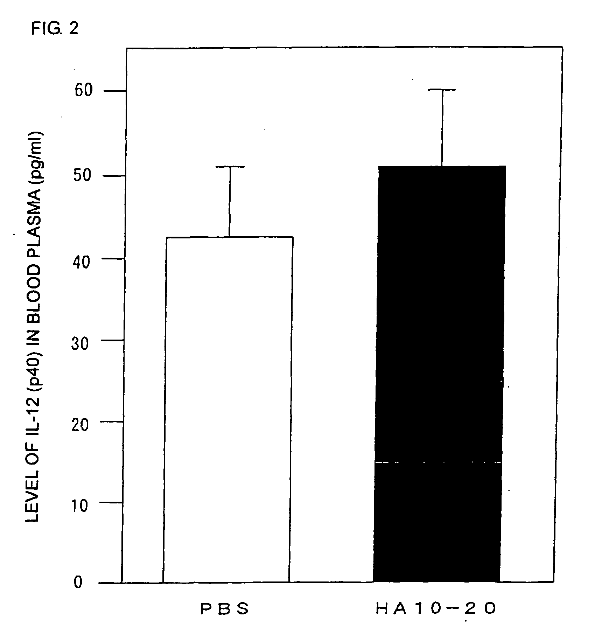

After completion of the administration, blood samples were collected from the orbital venous plexus, and concentration of IL-12 (p40) in blood plasma was measured using Mouse IL-12 (p40) ELISA Kit (manufactured by Endogen) to calculate its average value and standard deviation. The results are shown in Fig. 2.

Based on this result, it was shown that IL-12 production can be enhanced by oral administration of a low molecular weight sodium hyaluronate having a weight average molecular weight of about from 100,000 to 200,000.

### (2) Increase of serum IL-12 by HA30 administration

Four-weeks-old male C57BL/6 mice (Charles River Japan, Inc.) were purchased and divided into PBS administration group (n = 5) and HA30 administration group (n = 5).

A PBS solution containing 100 µg/ml HA30 was orally administered at a dose of 0.5 ml (about 2.5 mg/kg body weight) per once. The oral administration was carried out in the usual way similar to the above case. The administration was carried out once a day continuously for 13 days.

After completion of the administration, blood samples were collected from the orbital venous plexus, and concentration of IL-12 (p40) in blood plasma was measured by the same method described above to calculate its average value and standard deviation. The result (average value ± standard deviation) is shown below.
PBS administration group : 151.5 ± 18.4 (pg/ml)
HA30 administration group: 212.8 ± 23.6 (pg/ml)

In addition, this result was significant with p < 0.05 by Student's T-test.

Based on the above result, it was shown that IL-12 production can be significantly enhanced by oral administration of a low molecular weight sodium hyaluronate having a weight average molecular weight of about 300,000.

Thus, regarding the IL-12 production, the hyaluronan having a molecular weight of 600,000 showed a tendency to inhibit it slightly, the hyaluronan having a molecular weight of 840,000 inhibited it considerably, and the hyaluronan having a molecular weight of 2,700,000 showed a tendency to inhibit it though very slightly. On the other hand, the hyaluronan having a molecular weight of about from 100,000 to 300,000 conversely enhanced the IL-12 production. When these results are synthetically taken into consideration, it is considered that hyaluronan exerts the action to inhibit IL-12 expression within a range of molecular weight centering at 840,000 and extending plus and minus sides than that by a factor of about 300,000 (a molecular weight of from 600,000 to 1,200,000).

While the invention has been described in detail and with reference to specific embodiments thereof, it will be apparent to one of skill in the art that various changes and modifications can be made therein without departing from the spirit and scope thereof.

This application is based on Japanese application No. 2001-074077 filed on March 15, 2001 and Japanese application No. 2001-074078 filed on March 15, 2001, the entire contents of which are incorporated hereinto by reference.

### INDUSTRIAL APPLICABILITY

Particularly, since the inhibitor of the invention shows IL-12 expression inhibitory activity by its oral administration and a natural product is used as the material, its safety is also high and its availability is markedly high.

On the other hand, since the enhancer of the invention comprising a low molecular weight hyaluronan having a specified weight average molecular weight or a pharmaceutically acceptable salt thereof as an active ingredient shows an effect to enhance IL-12 production by its oral administration as is apparent also from the results of the above drug efficacy pharmacological tests, it is markedly useful for the treatment of animals which require enhancement of IL-12 production.

Particularly, since the enhancer of the invention uses a natural product as the material, its safety is also high and its availability is markedly high.

In addition, since the oral preparation of the present invention can be conveniently administered to patients by its oral administration, it is markedly useful.

## Claims

1. Use of hyaluronan or a pharmaceutically acceptable salt thereof having a weight average molecular weight of from 600,000 to 3,000,000 for preparing a medicament for the treatment or prevention of autoimmune diseases relating to activation of Th1, wherein the medicament is used for oral administration.

2. Use of hyaluronan or a pharmaceutically acceptable salt thereof having a weight average molecular weight of from 600,000 to 3,000,000 for preparing a medicament for the treatment or prevention of contact dermatitis, autoimmune uvea retinitis, allergic cerebrospinal meningitis, insulin-dependent diabetes mellitus, diabetes mellitus, Hashimoto disease, multiple sclerosis, rheumatic arthritis, Sjögren syndrome, Crohn disease, sarcoidosis, psoriasis, lipopolysaccharide-induced hepatic necrosis, crescentic glomerulonephritis, or systemic lupus erythematosus, wherein the medicament is used for oral administration.

3. The use according to claim 2, wherein the medicament is for the treatment or prevention of rheumatic arthritis or Crohn disease.

4. The use according to any one of claims 1 to 3, wherein the hyaluronan or the pharmaceutically acceptable salt thereof has a weight average molecular weight of from 600,000 to 1,200,000.

5. The use according to any one of claims 1 to 4, wherein the hyaluronan or the pharmaceutically acceptable salt thereof has a weight average molecular weight of from 700,000 to 1,200,000.

6. The use according to any one of claims 1 to 5, wherein the hyaluronan or the pharmaceutically acceptable salt thereof has a weight average molecular weight of from 700,000 to 1,100,000.

7. The use according to any one of claims 1 to 6, wherein the hyaluronan or the pharmaceutically acceptable salt thereof has a weight average molecular weight of from 800,000 to 1,100,000.

8. The use according to any one of claims 1 to 7, wherein the hyaluronan or the pharmaceutically acceptable salt thereof has a weight average molecular weight of from 800,000 to 1,000,000.

9. The use according to any one of claims 1 to 8, wherein the hyaluronan or the pharmaceutically acceptable salt thereof has a weight average molecular weight of from 800,000 to 950,000.

10. The use according to any one of claims 1 to 9, wherein the hyaluronan or the pharmaceutically acceptable salt thereof has a weight average molecular weight of from 800,000 to 900,000.

11. Use of hyaluronan or a pharmaceutically acceptable salt thereof having a weight average molecular weight of from 50,000 to 400,000 for preparing a medicament for the treatment or prevention of diseases caused by microbial infection, viral diseases or cancers for oral administration.

12. The use according to claim 11, wherein the hyaluronan or the pharmaceutically acceptable salt thereof has a weight average molecular weight of from 100,000 to 300,000.

13. Hyaluronan or a pharmaceutically acceptable salt thereof having a weight average molecular weight of from 600,000 to 3,000,000 for use in the treatment or prevention of autoimmune diseases relating to activation of Th1, wherein the hyaluronan or pharmaceutically acceptable salt thereof is used for oral administration.

14. Hyaluronan or a pharmaceutically acceptable salt thereof having a weight average molecular weight of from 600,000 to 3,000,000 for use in the treatment or prevention of contact dermatitis, autoimmune uvea retinitis, allergic cerebrospinal meningitis, insulin-dependent diabetes mellitus, diabetes mellitus, Hashimoto disease, multiple sclerosis, rheumatic arthritis, Sjögren syndrome, Crohn disease, sarcoidosis, psoriasis, lipopolysaccharide-induced hepatic necrosis, crescentic glomerulonephritis, or systemic lupus erythematosus, wherein the hyaluronan or pharmaceutically acceptable salt thereof is used for oral administration.

15. The hyaluronan or pharmaceutically acceptable salt thereof according to claim 14, which is for the treatment or prevention of rheumatic arthritis or Crohn disease.

16. The hyaluronan or pharmaceutically acceptable salt thereof according to any one of claims 13 to 15, wherein the hyaluronan or the pharmaceutically acceptable salt thereof has a weight average molecular weight of from 600,000 to 1,200,000.

17. The hyaluronan or pharmaceutically acceptable salt thereof according to any one of claims 13 to 16, wherein the hyaluronan or the pharmaceutically acceptable salt thereof has a weight average molecular weight of from 700,000 to 1,200,000.

18. The hyaluronan or pharmaceutically acceptable salt thereof according to any one of claims 13 to 17, wherein the hyaluronan or the pharmaceutically acceptable salt thereof has a weight average molecular weight of from 700,000 to 1,100,000.

19. The hyaluronan or pharmaceutically acceptable salt thereof according to any one of claims 13 to 18, wherein the hyaluronan or the pharmaceutically acceptable salt thereof has a weight average molecular weight of from 800,000 to 1,100,000.

20. The hyaluronan or pharmaceutically acceptable salt thereof according to any one of claims 13 to 19, wherein the hyaluronan or the pharmaceutically acceptable salt thereof has a weight average molecular weight of from 800,000 to 1,000,000.

21. The haluronan or pharmaceutically acceptable salt thereof according to any one of claims 13 to 20, wherein the hyaluronan or the pharmaceutically acceptable salt thereof has a weight average molecular weight of from 800,000 to 950,000.

22. The hyaluronan or pharmaceutically acceptable salt thereof according to any one of claims 13 to 21, wherein the hyaluronan or the pharmaceutically acceptable salt thereof has a weight average molecular weight of from 800,000 to 900,000.

23. Hyaluronan or a pharmaceutically acceptable salt thereof having a weight average molecular weight of from 50,000 to 400,000 for use in the treatment or prevention of diseases caused by microbial infection, viral diseases or cancers for oral administration.

24. The hyaluronan or pharmaceutically acceptable salt thereof according to claim 23, wherein the hyaluronan or the pharmaceutically acceptable salt thereof has a weight average molecular weight of from 100,000 to 300,000.

## Patentansprüche

1. Verwendung von Hyaluronsäure oder einem pharmazeutisch annehmbaren Salz davon mit einem gewichtsgemittelten Molekulargewicht von 600.000 bis 3.000.000 zum Herstellen eines Medikaments zur Behandlung oder Prävention von Autoimmunkrankheiten in Zusammenhang mit einer Th1-Aktivierung, worin das Medikament zur oralen Verabreichung verwendet wird.

2. Verwendung von Hyaluronsäure oder einem pharmazeutisch annehmbaren Salz davon mit einem gewichtsgemittelten Molekulargewicht von 600.000 bis 3.000.000 zum Herstellen eines Medikaments zur Behandlung oder Prävention von Kontaktdermatitis, autoimmuner uvealer Retinitis, allergischer Zerebrospinalmeningitis, insulinabhängigem Diabetes mellitus, Diabetes mellitus, Morbus Hashimoto, multipler Sklerose, rheumatischer Arthritis, Sjögren-Syndrom, Morbus Crohn, Sarkoidose, Psoriasis, Lipopolysaccharid-induzierter hepatischer Nekrose, halbmondförmiger Glomerulonephritis oder systemischem Lupus erythematosus, worin das Medikament zur oralen Verabreichung verwendet wird.

3. Verwendung gemäß Anspruch 2, worin das Medikament zur Behandlung oder Prävention von rheumatischer Arthritis oder Morbus Crohn ist.

4. Verwendung gemäß einem der Ansprüche 1 bis 3, worin die Hyaluronsäure oder das pharmazeutisch annehmbare Salz davon ein gewichtsgemitteltes Molekulargewicht von 600.000 bis 1.200.000 aufweist.

5. Verwendung gemäß einem der Ansprüche 1 bis 4, worin die Hyaluronsäure oder das pharmazeutisch annehmbare Salz davon ein gewichtsgemitteltes Molekulargewicht von 700.000 bis 1.200.000 aufweist.

6. Verwendung gemäß einem der Ansprüche 1 bis 5, worin die Hyaluronsäure oder das pharmazeutisch annehmbare Salz davon ein gewichtsgemitteltes Molekulargewicht von 700.000 bis 1.100.000 aufweist.

7. Verwendung gemäß einem der Ansprüche 1 bis 6, worin die Hyaluronsäure oder das pharmazeutisch annehmbare Salz davon ein gewichtsgemitteltes Molekulargewicht von 800.000 bis 1.100.000 aufweist.

8. Verwendung gemäß einem der Ansprüche 1 bis 7, worin die Hyaluronsäure oder das pharmazeutisch annehmbare Salz davon ein gewichtsgemitteltes Molekulargewicht von 800.000 bis 1.000.000 aufweist.

9. Verwendung gemäß einem der Ansprüche 1 bis 8, worin die Hyaluronsäure oder das pharmazeutisch annehmbare Salz davon ein gewichtsgemitteltes Molekulargewicht von 800.000 bis 950.000 aufweist.

10. Verwendung gemäß einem der Ansprüche 1 bis 9, worin die Hyaluronsäure oder das pharmazeutisch annehmbare Salz davon ein gewichtsgemitteltes Molekulargewicht von 800.000 bis 900.000 aufweist.

11. Verwendung von Hyaluronsäure oder einem pharmazeutisch annehmbaren Salz davon mit einem gewichtsgemittelten Molekulargewicht von 50.000 bis 400.000 zum Herstellen eines Medikaments zur Behandlung oder Prävention von Krankheiten, die durch eine mikrobielle Infektion, virale Erkrankungen oder Krebs verursacht werden, zur oralen Verabreichung.

12. Verwendung gemäß Anspruch 11, worin die Hyaluronsäure oder das pharmazeutisch annehmbare Salz davon ein gewichtsgemitteltes Molekulargewicht von 100.000 bis 300.000 aufweist.

13. Hyaluronsäure oder ein pharmazeutisch annehmbares Salz davon mit einem gewichtsgemittelten Molekulargewicht von 600.000 bis 3.000.000 zur Verwendung in der Behandlung oder Prävention von Autoimmunkrankheiten in Zusammenhang mit einer Th1-Aktivierung, worin die Hyaluronsäure oder das pharmazeutisch annehmbare Salz davon für eine orale Verabreichung verwendet wird.

14. Hyaluronsäure oder ein pharmazeutisch annehmbares Salz davon mit einem gewichtsgemittelten Molekulargewicht von 600.000 bis 3.000.000 zur Verwendung in der Behandlung oder Prävention von Kontaktdermatitis, autoimmuner uvealer Retinitis, allergischer Zerebrospinalmeningitis, insulinabhängigem Diabetes mellitus, Diabetes mellitus, Morbus Hashimoto, multipler Sklerose, rheumatischer Arthritis, Sjögren-Syndrom, Morbus Crohn, Sarkoidose, Psoriasis, Lipopolysaccharid-induzierter hepatischer Nekrose, halbmondförmiger Glomerulonephritis oder systemischem Lupus erythematosus, worin die Hyaluronsäure oder das pharmazeutisch annehmbare Salz davon zur oralen Verabreichung verwendet wird.

15. Hyaluronsäure oder ein pharmazeutisch annehmbares Salz davon gemäß Anspruch 14, worin die Hyaluronsäure oder das pharmazeutisch annehmbare Salz davon zur Behandlung oder Prävention von rheumatischer Arthritis oder Morbus Crohn ist.

16. Hyaluronsäure oder ein pharmazeutisch annehmbares Salz davon gemäß einem der Ansprüche 13 bis 15, worin die Hyaluronsäure oder das pharmazeutisch annehmbare Salz davon ein gewichtsgemitteltes Molekulargewicht von 600.000 bis 1.200.000 aufweist.

17. Hyaluronsäure oder ein pharmazeutisch annehmbares Salz davon gemäß einem der Ansprüche 13 bis 16, worin die Hyaluronsäure oder das pharmazeutisch annehmbare Salz davon ein gewichtsgemitteltes Molekulargewicht von 700.000 bis 1.200.000 aufweist.

18. Hyaluronsäure oder ein pharmazeutisch annehmbares Salz davon gemäß einem der Ansprüche 13 bis 17, worin die Hyaluronsäure oder das pharmazeutisch annehmbare Salz davon ein gewichtsgemitteltes Molekulargewicht von 700.000 bis 1.100.000 aufweist.

19. Hyaluronsäure oder ein pharmazeutisch annehmbares Salz davon gemäß einem der Ansprüche 13 bis 18, worin die Hyaluronsäure oder das pharmazeutisch annehmbare Salz davon ein gewichtsgemitteltes Molekulargewicht von 800.000 bis 1.100.000 aufweist.

20. Hyaluronsäure oder ein pharmazeutisch annehmbares Salz davon gemäß einem der Ansprüche 13 bis 19, worin die Hyaluronsäure oder das pharmazeutisch annehmbare Salz davon ein gewichtsgemitteltes Molekulargewicht von 800.000 bis 1.000.000 aufweist.

21. Hyaluronsäure oder ein pharmazeutisch annehmbares Salz davon gemäß einem der Ansprüche 13 bis 20, worin die Hyaluronsäure oder das pharmazeutisch annehmbare Salz davon ein gewichtsgemitteltes Molekulargewicht von 800.000 bis 950.000 aufweist.

22. Hyaluronsäure oder ein pharmazeutisch annehmbares Salz davon gemäß einem der Ansprüche 13 bis 21, worin die Hyaluronsäure oder das pharmazeutisch annehmbare Salz davon ein gewichtsgemitteltes Molekulargewicht von 800.000 bis 900.000 aufweist.

23. Hyaluronsäure oder ein pharmazeutisch annehmbares Salz davon mit einem gewichtsgemittelten Molekulargewicht von 50.000 bis 400.000 zur Verwendung in der Behandlung oder Prävention von Krankheiten, die durch eine mikrobielle Infektion, virale Krankheiten oder Krebs verursacht werden, zur oralen Verabreichung.

24. Hyaluronsäure oder ein pharmazeutisch annehmbares Salz davon gemäß Anspruch 23, worin die Hyaluronsäure oder das pharmazeutisch annehmbare Salz davon ein gewichtsgemitteltes Molekulargewicht von 100.000 bis 300.000 aufweist.

## Revendications

1. Utilisation de hyaluronan ou d'un sel pharmaceutiquement acceptable de celui-ci ayant une masse moléculaire moyenne en masse de 600 000 à 3 000 000 pour la préparation d'un médicament destiné au traitement ou à la prévention de maladies autoimmunes en relation avec l'activation de Th1, où le médicament est utilisé pour l'administration orale.

2. Utilisation de hyaluronan ou d'un sel pharmaceutiquement acceptable de celui-ci ayant une masse moléculaire moyenne en masse de 600 000 à 3 000 000 pour la préparation d'un médicament destiné au traitement ou à la prévention de la dermatite de contact, de la rétinite uvéale autoimmune, de la méningite cérébrospinale allergique, du diabète sucré insulino-dépendant, du diabète sucré, de la maladie d'Hashimoto, de la sclérose en plaques, de la polyarthrite rhumatoïde, du syndrome de Sjögren, de la maladie de Crohn, de la sarcoïdose, du psoriasis, de la nécrose hépatique induite par les lipopolysaccharides, de la glomérulonéphrite maligne ou du lupus érythémateux disséminé, où le médicament est utilisé pour l'administration orale.

3. Utilisation selon la revendication 2, où le médicament est destiné au traitement ou à la prévention de la polyarthrite rhumatoïde ou de la maladie de Crohn.

4. Utilisation selon l'une quelconque des revendications 1 à 3, où le hyaluronan ou son sel pharmaceutiquement acceptable a une masse moléculaire en masse de 600 000 à 1 200 000.

5. Utilisation selon l'une quelconque des revendications 1 à 4, où le hyaluronan ou son sel pharmaceutiquement acceptable a une masse moléculaire moyenne en masse de 700 000 à 1 200 000.

6. Utilisation selon l'une quelconque des revendications 1 à 5, où le hyaluronan ou son sel pharmaceutiquement acceptable a une masse moléculaire moyenne en masse de 700 000 à 1 100 000.

7. Utilisation selon l'une quelconque des revendications 1 à 6, où le hyaluronan ou son sel pharmaceutiquement acceptable a une masse moléculaire moyenne en masse de 800 000 à 1 100 000.

8. Utilisation selon l'une quelconque des revendications 1 à 7, où le hyaluronan ou son sel pharmaceutiquement acceptable a une masse moléculaire moyenne en masse de 800 000 à 1 000 000.

9. Utilisation selon l'une quelconque des revendications 1 à 8, où le hyaluronan ou son sel pharmaceutiquement acceptable a une masse moléculaire moyenne en masse de 800 000 à 950 000.

10. Utilisation selon l'une quelconque des revendications 1 à 9, où le hyaluronan ou son sel pharmaceutiquement acceptable a une masse moléculaire moyenne en masse de 800 000 à 900 000.

11. Utilisation de hyaluronan ou d'un sel pharmaceutiquement acceptable de celui-ci ayant une masse moléculaire moyenne en masse de 50 000 à 400 000 pour la préparation d'un médicament destiné au traitement ou à la prévention de maladies provoquées par une infection microbienne, de maladies virales ou de cancers, pour l'administration orale.

12. Utilisation selon la revendication 11, où le hyaluronan ou son sel pharmaceutiquement acceptable a une masse moléculaire moyenne en masse de 100 000 à 300 000.

13. Hyaluronan ou sel pharmaceutiquement acceptable de celui-ci ayant une masse moléculaire moyenne en masse de 600 000 à 3 000 000 pour utilisation pour le traitement ou la prévention de maladies autoimmunes en relation avec l'activation de Th1, où le hyaluronan ou son sel pharmaceutiquement acceptable est utilisé pour l'administration orale.

14. Hyaluronan ou sel pharmaceutiquement acceptable de celui-ci ayant une masse moléculaire moyenne en masse de 600 000 à 3 000 000 pour utilisation dans le traitement ou la prévention de la dermatite de contact, de la rétinite uvéale autoimmune, de la méningite cérébrospinale allergique, du diabète sucré insulino-dépendant, du diabète sucré, de la maladie de Hashimoto, de la sclérose en plaques, de la polyarthrite rhumatoïde, du syndrome de Sjögren, de la maladie de Crohn, de la sarcoïdose, du psoriasis, de la nécrose hépatique induite par les lipopolysaccharides, de la glomérulonéphrite maligne ou du lupus érythémateux disséminé, où le hyaluronan ou son sel pharmaceutiquement acceptable est utilisé pour l'administration orale.

15. Hyaluronan ou sel pharmaceutiquement acceptable de celui-ci selon la revendication 14, qui est destiné au traitement ou à la prévention de la polyarthrite rhumatoïde ou de la maladie de Crohn.

16. Hyaluronan ou sel pharmacetiquement acceptable de celui-ci selon l'une quelconque des revendications 13 à 15, où le hyaluronan ou son sel pharmaceutiquement acceptable a une masse moléculaire moyenne en masse de 600 000 à 1 200 000.

17. Hyaluronan ou sel pharmaceutiquement acceptable de celui-ci selon l'une quelconque des revendications 13 à 16, où le hyaluronan ou son sel pharmaceutiquement acceptable a une masse moléculaire moyenne en masse de 700 000 à 1 200 000.

18. Hyaluronan ou sel pharmaceutiquement acceptable de celui-ci selon l'une quelconque des revendications 13 à 17, où le hyaluronan ou son sel pharmaceutiquement acceptable a une masse moléculaire moyenne en masse de 700 000 à 1 100 000.

19. Hyaluronan ou sel pharmaceutiquement acceptable de celui-ci selon l'une quelconque des revendications 13 à 18, où le hyaluronan ou son sel pharmaceutiquement acceptable a une masse moléculaire moyenne en masse de 800 000 à 1 100 000

20. Hyaluronan ou sel pharmaceutiquement acceptable de celui-ci selon l'une quelconque des revendications 13 à 19, où le hyaluronan ou son sel pharmaceutiquement acceptable a une masse moléculaire moyenne en masse de 800 000 à 1 000 000.

21. Hyaluronan ou sel pharmaceutiquement acceptable de celui-ci selon l'une quelconque des revendications 13 à 20, où le hyaluronan ou son sel pharmaceutiquement acceptable a une masse moléculaire moyenne en masse de 800 000 à 950 000.

22. Hyaluronan ou sel pharmaceutiquement acceptable de celui-ci selon l'une quelconque des revendications 13 à 21, où le hyaluronan ou son sel pharmaceutiquement acceptable a une masse moléculaire moyenne en masse de 800 000 à 900 000.

23. Hyaluronan ou sel pharmaceutiquement acceptable de celui-ci ayant une masse moléculaire moyenne en masse de 50 000 à 400 000 pour utilisation dans le traitement ou la prévention de maladies provoquées par une infection microbienne, de maladies virales ou de cancers, pour administration orale.

24. Hyaluronan ou sel pharmaceutiquement acceptable de celui-ci selon la revendication 23, où le hyaluronan ou son sel pharmaceutiquement acceptable a une masse moléculaire moyenne en masse de 100 000 à 300 000.
